# EUROPEAN PATENT APPLICATION

(11) **EP 4 471 193 A2**
(43) Date of publication of application: **04.12.2024**
(21) Application number: 24171186.0
(22) Date of filing: 19.04.2024
(51) Int. Cl.: D01D 5/16, D01D 10/02, D02J 1/22, D02J 13/00

(54) **APPARATUS FOR PRODUCING FULLY DRAWN YARN**

(30) Priority: 31.05.2023 JP 2023089999
(71) Applicant: TMT Machinery, Inc., Osaka-shi, Osaka 541-0041 (JP)
(72) Inventor: TSUJI, Takahiro, Kyoto-shi, Kyoto, 612-8686 (JP); HAYASHI, Yasushi, Kyoto-shi, Kyoto, 612-8686 (JP); HASHIMOTO, Kinzo, Kyoto-shi, Kyoto, 612-8686 (JP)
(74) Representative: Hoffmann Eitle

(57) **Abstract**

An apparatus for producing a fully drawn yarn includes: godet rollers 31 to 33 being preliminary heating rollers, and godet rollers 34 and 35 which are heat-setting rollers arranged downstream in a yarn running direction of the godet rollers 31 to 33, heat a yarn Y at a higher temperature than the godet rollers 31 to 33, and feed the yarn Y at a yarn feeding speed higher than that of the godet rollers 31 to 33. A tension sensor 5 that measures a tension of the yarn Y and a heater 6 that heats the yarn Y are arranged between a guide roller 13 and a guide roller 14 adjacent in the yarn running direction. The tension sensor 5 measures the tension of the yarn Y subjected to drawing processing between the godet roller 33 and the godet roller 34 on the downstream in the yarn running direction of the godet roller 34 located on the most upstream in the yarn running direction of the heat-setting roller.

## Description

### BACKGROUND OF THE INVENTION

The present invention relates to an apparatus for producing a fully drawn yarn subjected to drawing processing.

The dyeing characteristics (for example, the dyeing density, the likelihood of the dyeing unevenness, and the like) of a fully drawn yarn (FDY) subjected to the drawing processing vary depending on the degree of molecular orientation and the degree of crystallinity. The dyeing characteristics of the yarn greatly affect the commercial value of the yarn. Therefore, a test for determining the dyeing characteristics of the yarn in advance has been conventionally performed. As disclosed in JP 2015-212924 A, in such a test, a sample obtained by tubular knitting of a yarn to be determined is actually dyed, and the dyeing characteristics are determined based on the dyed state of the sample.

### SUMMARY OF THE INVENTION

The test for determining the dyeing characteristics is performed on all packages formed by winding the fully drawn yarn around a bobbin in the apparatus for producing a fully drawn yarn. That is, after the packages are formed in the apparatus for producing a fully drawn yarn, a step of actually dyeing the yarn is carried out for all the packages. Therefore, the number of steps of the test for determining the dyeing characteristics becomes enormous. Therefore, there arises a problem that it takes a lot of time to determine the dyeing characteristics.

An object of the present invention is to provide an apparatus for producing a fully drawn yarn capable of determining dyeing characteristics in a short time.

An apparatus for producing a fully drawn yarn according to a first invention includes: a preliminary heating roller that heats a yarn; and at least one heat-setting roller that is arranged downstream in a yarn running direction of the preliminary heating roller, heats the yarn at a higher temperature than the preliminary heating roller, and feeds the yarn at a yarn feeding speed higher than a yarn feeding speed of the preliminary heating roller, the apparatus being configured to produce a fully drawn yarn by subjecting a yarn spun from a spinning apparatus to drawing processing, the apparatus further including: a plurality of feed rollers that include the preliminary heating roller and the at least one heat-setting roller and feed the yarn; and a tension sensor that is arranged between a first roller and a second roller, which are two feed rollers adjacent in the yarn running direction among the plurality of feed rollers, and measures a tension of the yarn, in which at least one of the first roller and the second roller located upstream in the yarn running direction is capable of heating the yarn, and in which the tension sensor measures a tension of the yarn subjected to drawing processing between the preliminary heating roller and a most upstream heat-setting roller downstream in the yarn running direction of the most upstream heat-setting roller located the most upstream in the yarn running direction of the at least one heat-setting roller.

In the present invention, the tension sensor can measure the tension of the fully drawn yarn in a state of being heated by at least one of the first roller and the second roller located upstream in the yarn running direction. Here, the oriented amorphous part in which the orientation in the fully drawn yarn is advanced but not crystallized is less likely to be dyed than the non-oriented amorphous part in which the orientation and the crystallization are not performed. In addition, the oriented amorphous part has a characteristic that shrinkage force increases during heating. Therefore, it can be determined that the fully drawn yarn having a larger tension measured in a heated state has a higher proportion occupied by the oriented amorphous part than the non-oriented amorphous part, and has a characteristic of being less likely to be dyed. That is, the dyeing characteristics of the fully drawn yarn can be determined based on the tension of the yarn measured by the tension sensor without requiring the step of actually dyeing the yarn. The tension sensor provided in the apparatus for processing the fully drawn yarn can acquire tension for determining the dyeing characteristics of the fully drawn yarn in the process of processing the fully drawn yarn. Therefore, the dyeing characteristics can be determined in a short time.

In the apparatus for producing a fully drawn yarn according to a second invention, in the first invention, one of the first roller and the second roller arranged upstream in the yarn running direction is the heat-setting roller.

In the present invention, the tension sensor can measure the tension of the yarn in a state of being heated by the heat-setting roller. Therefore, the tension of the yarn in the heated state can be measured without providing an extra roller for heating the yarn.

In the apparatus for producing a fully drawn yarn according to a third invention, in the second invention, at least two heat-setting rollers are provided, and the first roller and the second roller are the heat-setting rollers.

In the present invention, the tension of the yarn in a state of being reliably heated by the two heat-setting rollers can be measured without providing an extra roller for heating the yarn.

An apparatus for producing a fully drawn yarn according to a fourth aspect of the present invention includes: a preliminary heating roller that heats a yarn; and at least one heat-setting roller that is arranged downstream in a yarn running direction of the preliminary heating roller, heats the yarn at a higher temperature than the preliminary heating roller, and feeds the yarn at a yarn feeding speed higher than a yarn feeding speed of the preliminary heating roller, the apparatus being configured to produce a fully drawn yarn by subjecting a yarn spun from a spinning apparatus to drawing processing, the apparatus further including: a plurality of feed rollers that include the preliminary heating roller and the at least one heat-setting roller and feed the yarn; a tension sensor that is arranged between a first roller and a second roller, which are two feed rollers adjacent in the yarn running direction among the plurality of feed rollers, and measures a tension of the yarn; and a heater that is arranged between the first roller and the second roller and heats the yarn, in which the tension sensor measures a tension of the yarn subjected to drawing processing between the preliminary heating roller and a most upstream heat-setting roller downstream in the yarn running direction of the most upstream heat-setting roller located the most upstream in the yarn running direction of the at least one heat-setting roller.

The "heater that is arranged between the first roller and the second roller and heats the yarn" is a heater arranged outside the first roller and the second roller and between the first roller and the second roller. That is, the "heater" here does not include a heater incorporated in the first roller or the second roller.

In the present invention, the tension of the fully drawn yarn in a state of being heated by the heater can be measured by the tension sensor. Here, the oriented amorphous part in which the orientation in the fully drawn yarn is advanced but not crystallized is less likely to be dyed than the non-oriented amorphous part in which the orientation and the crystallization are not performed. In addition, the oriented amorphous part has a characteristic that shrinkage force increases during heating. Therefore, it can be determined that the fully drawn yarn having a larger tension measured in a heated state has a higher proportion occupied by the oriented amorphous part than the non-oriented amorphous part, and has a characteristic of being less likely to be dyed. That is, the dyeing characteristics of the fully drawn yarn can be determined based on the tension of the yarn measured by the tension sensor without requiring the step of actually dyeing the yarn. The tension sensor provided in the apparatus for processing the fully drawn yarn can acquire tension for determining the dyeing characteristics of the fully drawn yarn in the process of processing the fully drawn yarn. Therefore, the dyeing characteristics can be determined in a short time.

In the apparatus for producing a fully drawn yarn according to a fifth invention, in the first to fourth inventions, the second roller is arranged downstream of the first roller in the yarn running direction, a yarn feeding speed of the second roller is set to be higher than a yarn feeding speed of the first roller, and the tension sensor measures a tension of the yarn in a state of being stretched between the first roller and the second roller.

In the present invention, the tension of the fully drawn yarn in the stretched state can be measured by the tension sensor. Here, in addition to the oriented amorphous part, the oriented crystalline part in which molecular orientation in the fully drawn yarn is advanced and crystallization is also advanced is less likely to be dyed than the non-oriented amorphous part. In addition, the oriented crystalline part has a characteristic that the resistance force increases at the time of stretching. Therefore, it can be determined that the fully drawn yarn having a larger tension measured in the stretched state while being heated has a higher proportion occupied by the oriented amorphous part and the oriented crystalline part than the non-oriented amorphous part, and has a characteristic of being less likely to be dyed. Therefore, the dyeing characteristics of the fully drawn yarn can be more accurately determined.

### BRIEF DESCRIPTION OF THE DRAWINGS

FIG. 1 is a schematic view showing a spun yarn take-up machine according to a first embodiment;
FIG. 2 is a block diagram showing an electrical configuration of the spun yarn take-up machine shown in FIG. 1;
FIGS. 3A to 3C are views for explaining a molecular structure of a fully drawn yarn, in which FIG. 3A shows a non-oriented amorphous part, FIG. 3B shows an oriented amorphous part, and FIG. 3C shows an oriented crystalline part;
FIG. 4 is a graph showing tension measurement results of three types of fully drawn yarns;
FIGS. 5A, 5B, and 5C are stained images of samples each made of three types of fully drawn yarns;
FIG. 6 is a table showing measurement results of tension and confirmation results of dyeing characteristics of nine types of fully drawn yarns;
FIG. 7 is a graph showing the relationship between an average value of measured values of the tensions of the nine types of fully drawn yarns and the L value and the b value of color measurement; and
FIG. 8 is a schematic view showing a spun yarn take-up machine according to a second embodiment.

### DESCRIPTION OF THE PREFERRED EMBODIMENTS

### <First Embodiment>

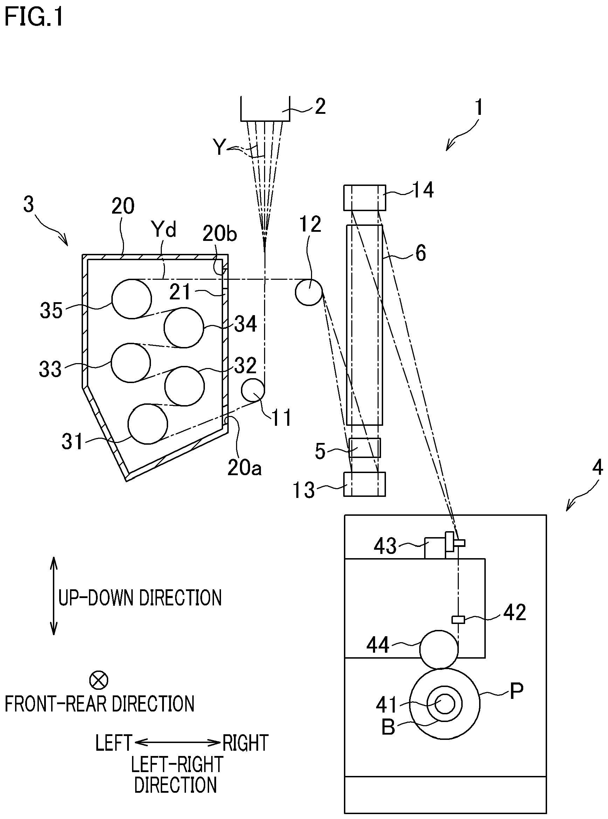

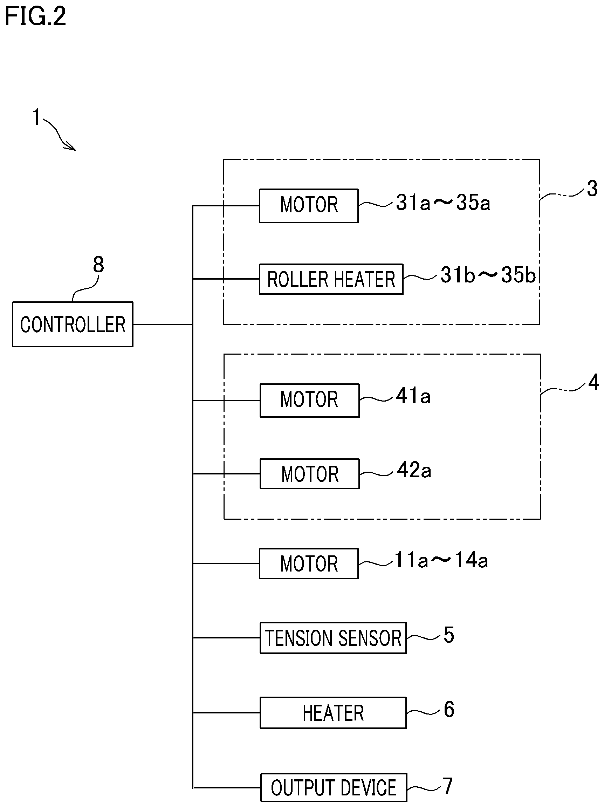

Hereinafter, a spun yarn take-up machine according to a preferred first embodiment of the present invention (corresponding to an "apparatus for producing a fully drawn yarn" of the present invention) will be described with reference to FIGS. 1 and 2. Note that a direction perpendicular to the paper surface of FIG. 1 is a front-rear direction, and a left-right direction of the paper surface is a left-right direction. A direction orthogonal to both the front-rear direction and the left-right direction is defined as an up-down direction in which gravity acts.

As shown in FIGS. 1 and 2, the spun yarn take-up machine 1 mainly includes a spun yarn drawing apparatus 3, a take-up winder 4, guide rollers 11 to 14, a tension sensor 5, a heater 6, an output device 7, and a controller 8.

The spun yarn take-up machine 1 is configured to form a plurality of packages P by drawing a plurality of yarns Y spun from the spinning apparatus 2 by the spun yarn drawing apparatus 3 and then winding the plurality of yarns Y by the take-up winder 4. The spinning apparatus 2 continuously spins a molten fibrous material such as polyester to produce the plurality of yarns Y. The plurality of yarns Y spun from the spinning apparatus 2 are sent to the spun yarn drawing apparatus 3 via the guide roller 11. The plurality of yarns Y (fully drawn yarn Yd) drawn by the spun yarn drawing apparatus 3 are fed to the take-up winder 4 by the guide rollers 12 to 14.

The tension sensor 5 measures the tension of the fully drawn yarn Yd subjected to drawing processing. The heater 6 heats the fully drawn yarn Yd whose tension is measured by the tension sensor 5. The measurement result of the tension of the fully drawn yarn Yd by the tension sensor 5 is output to the output device 7. The controller 8 controls the operation of the entire spun yarn take-up machine 1.

The spun yarn drawing apparatus 3 is an apparatus that heats and draws the plurality of yarns Y, and is arranged below the spinning apparatus 2. The spun yarn drawing apparatus 3 includes a plurality of godet rollers 31 to 35 accommodated inside a thermal insulation box 20. A yarn inlet 20a and a yarn outlet 20b are formed on a side wall 21 on one side (right side) in the left-right direction of the thermal insulation box 20. The yarn inlet 20a is an inlet for introducing the plurality of yarns Y into the thermal insulation box 20. The yarn outlet 20b is an outlet for leading out the plurality of yarns Y from the inside to the outside of the thermal insulation box 20. The yarn inlet 20a is formed at the lower end portion of the side wall 21. The yarn outlet 20b is formed at the upper end portion of the side wall 21.

Each of the godet rollers 31 to 35 is a roller that feeds the yarn Y in a yarn running direction from the yarn inlet 20a toward the yarn outlet 20b of the thermal insulation box 20 while heating the yarn Y. The rotation axes of the godet rollers 31 to 35 are parallel to each other. The godet rollers 31 to 35 are each arranged in such a posture that its rotation axis is parallel to the front-rear direction. The godet rollers 31 to 35 are arranged in this order from the upstream in the yarn running direction. The plurality of yarns Y are wound around each of the godet rollers 31 to 35 at a winding angle of less than 360 degrees.

The godet rollers 31 to 35 are rotationally driven by motors 31a to 35a (see FIG. 2), respectively. The operation of the motors 31a to 35a is controlled by the controller 8. The yarn feeding speed of each of the godet rollers 31 to 35 is controlled by the controller 8. Here, the yarn feeding speed of the godet roller 31 is V1, the yarn feeding speed of the godet roller 32 is V2, the yarn feeding speed of the godet roller 33 is V3, the yarn feeding speed of the godet roller 34 is V4, and the yarn feeding speed of the godet roller 35 is V5. The controller 8 performs control such that V1 = V2 = V3 < V4 = V5. That is, the godet rollers 34 and 35 feed the yarn Y at a yarn feeding speed faster than the yarn feeding speeds of the godet rollers 31 to 33.

The godet rollers 31 to 35 incorporate roller heaters 31b to 35b (see FIG. 2) for heating the roller surface, respectively. The heating temperature of each of the roller heaters 31b to 35b is controlled by the controller 8.

Among the godet rollers 31 to 35, three godet rollers 31 to 33 located upstream in the yarn running direction are preliminary heating rollers that heat the yarn Y before drawing. The controller 8 controls the heating temperatures of the roller heaters 31b to 33b so that the roller surface temperatures of the godet rollers 31 to 33 become a temperature (for example, about 80 °C to 100 °C) equal to or higher than the glass transition temperature of the yarn Y. Among the godet rollers 31 to 35, two godet rollers 34 and 35 located downstream in the yarn running direction are heat-setting rollers for thermally setting the plurality of drawn yarns Y. The controller 8 controls the heating temperatures of the heaters 34b and 35b so that the roller surface temperatures of the godet rollers 34 and 35 are higher than the roller surface temperatures of the godet rollers 31 to 33 (for example, about 130 °C to 150 °C) . That is, the godet rollers 34 and 35 heat the yarn Y at a higher temperature than the godet rollers 31 to 33.

The plurality of yarns Y introduced into the thermal insulation box 20 through the yarn inlet 20a are first preheated to a drawable temperature while being fed by the godet rollers 31 to 33. The plurality of preheated yarns Y are drawn between the godet roller 33 and the godet roller 34. The godet roller 33 is a godet roller located on the most downstream side in the yarn running direction among the three godet rollers 31 to 33 which are preliminary heating rollers. The godet roller 34 is a godet roller located on the most upstream side in the yarn running direction among the two godet rollers 34 and 35 which are heat-setting rollers. That is, the godet roller 34 corresponds to "most upstream heat-setting roller" of the present invention. Thereafter, the plurality of yarns Y are further heated to a high temperature while being fed by the godet rollers 34 and 35, and the drawn state is thermally fixed. The plurality of yarns Y (fully drawn yarns Yd) drawn in this manner are led out of the thermal insulation box 20 through the yarn outlet 20b.

The take-up winder 4 is an apparatus that winds the plurality of yarns Y, and is arranged below the spun yarn drawing apparatus 3. The take-up winder 4 mainly includes a bobbin holder 41, a plurality of traverse guides 42, a plurality of fulcrum guides 43, and a contact roller 44.

The bobbin holder 41 has a cylindrical shape extending in the front-rear direction, and is rotationally driven by a motor 41a (see FIG. 2). The bobbin holder 41 is mounted with the plurality of bobbins B arranged in the axial direction thereof. The plurality of traverse guides 42 and the plurality of fulcrum guides 43 are respectively provided corresponding to the plurality of bobbins B. Each traverse guide 42 is reciprocated in the front-rear direction by a driving force from a motor 42a (see FIG. 2), and traverses the yarn Y hooked to the corresponding fulcrum guide 43.

The take-up winder 4 rotates the bobbin holder 41 to simultaneously wind the plurality of yarns Y around the plurality of bobbins B so as to produce the plurality of packages P. The contact roller 44 comes into contact with the surfaces of the plurality of packages P to apply a predetermined contact pressure, and adjusts the shapes of the packages P.

The guide roller 11 is arranged in such a posture that its rotation axis is parallel to the front-rear direction. The guide roller 11 is arranged on the right side of the thermal insulation box 20. The guide roller 11 is arranged in proximity to the yarn inlet 20a of the thermal insulation box 20. The guide roller 11 introduces the plurality of yarns Y into the thermal insulation box 20 through the yarn inlet 20a.

The guide roller 12 is arranged in such a posture that its rotation axis is parallel to the front-rear direction. The guide roller 12 is arranged on the right side of the thermal insulation box 20. The guide roller 12 is arranged at a position facing the yarn outlet 20b of the thermal insulation box 20 in the left-right direction. The plurality of yarns Y led out from the yarn outlet 20b to the outside of the thermal insulation box 20 are wound around the guide roller 12.

Both the guide rollers 13 and 14 are arranged in such a posture that its rotation axis is parallel to the left-right direction. The guide rollers 13 and 14 are both arranged on the right side of the thermal insulation box 20. The guide roller 13 is arranged below the guide roller 12. The plurality of yarns Y fed from the guide roller 12 are wound around the guide roller 13. The guide roller 13 feeds the plurality of yarns Y to the guide roller 14. The guide roller 14 is arranged behind and above the guide roller 13. The plurality of yarns Y fed from the guide roller 13 are wound around the guide roller 14. The guide roller 14 feeds the plurality of yarns Y to the take-up winder 4.

The guide rollers 11 to 14 are rotationally driven by motors 11a to 14a (see FIG. 2), respectively. The operation of the motors 11a to 14a is controlled by the controller 8. The yarn feeding speed of each of the guide rollers 11 to 14 is controlled by the controller 8. Here, the yarn feeding speed of the guide roller 12 is V6, the yarn feeding speed of the guide roller 13 is V7, and the yarn feeding speed of the guide roller 14 is V8. The controller 8 performs control such that V4 (yarn feeding speed of godet roller 34) = V5 (yarn feeding speed of godet roller 35) = V6 = V7 < V8. The difference between the yarn feeding speed (V7) of the guide roller 13 and the yarn feeding speed (V8) of the guide roller 14 is adjusted such that the plurality of yarns Y between the guide roller 13 and the guide roller 14 are in a state of being stretched by the deformation amount within the elastic range. In the present embodiment, none of the guide rollers 11 to 14 has a heater therein.

The five godet rollers 31 to 35 of the spun yarn drawing apparatus 3 and the guide rollers 11 to 14 correspond to "plurality of feed rollers" of the present invention.

The tension sensor 5 measures the tension of the yarn Y (fully drawn yarn Yd) subjected to the drawing processing between the godet roller 33 and the godet roller 34 at the downstream in the yarn running direction of the godet roller 34. The tension sensor 5 is arranged between the guide roller 13 and the guide roller 14, which are two rollers adjacent in the yarn running direction. Here, the guide roller 14 is arranged downstream of the guide roller 13 in the yarn running direction. As described above, the yarn feeding speed (V8) by the guide roller 14 is set to be faster than the yarn feeding speed (V7) by the guide roller 13. That is, the guide roller 13 corresponds to "first roller" of the present invention, and the guide roller 14 corresponds to "second roller" of the present invention.

The heater 6 is arranged between the guide roller 13 and the guide roller 14. In the present embodiment, the heater 6 is arranged downstream of the tension sensor 5 in the yarn running direction. The heater 6 may be arranged upstream of the tension sensor 5 in the yarn running direction.

The heating temperature of the heater 6 is controlled by the controller 8. The controller 8 controls the heating temperature of the heater 6 to be 120 °C to 140 °C. More specifically, the controller 8 controls the temperature of the fully drawn yarn Yd heated by the heater 6 to be 120 °C to 140 °C. The heating temperature of the heater 6 is set to a temperature lower than the heat fixation temperatures (roller surface temperatures (about 130 to 150 °C) of godet rollers 34 and 35 which are heat-setting rollers) at the time of producing the fully drawn yarn Yd. Therefore, the molecular structure of the fully drawn yarn Yd can be prevented from being affected by the heating by the heater 6. The polyester yarn Y is usually dyed under high temperature of about 130 °C to 135 °C. Therefore, by setting the heating temperature of the heater 6 to 120 °C to 140 °C, the tension of the fully drawn yarn Yd at the temperature at the time of dyeing can be measured.

The tension sensor 5 measures the tension of the fully drawn yarn Yd in a state of being heated by the heater 6. As described above, due to the difference in yarn feeding speed between the guide roller 13 and the guide roller 14, the fully drawn yarn Yd between the guide roller 13 and the guide roller 14 is stretched by the deformation amount within the elastic range. Therefore, the tension sensor 5 measures the tension of the fully drawn yarn Yd in a state of being stretched by the deformation amount within the elastic range.

The tension sensor 5 is arranged on each of the plurality of fully drawn yarns Yd running between the guide roller 13 and the guide roller 14. That is, the tensions of the plurality of fully drawn yarns Yd can be measured by the plurality of tension sensors 5, respectively. In the present embodiment, each tension sensor 5 measures the tension at a plurality of positions in the longitudinal direction of the fully drawn yarn Yd wound into one package P. The measurement of the tension by each tension sensor 5 may be performed at least at one position in the longitudinal direction of the fully drawn yarn Yd wound into one package P. The tension of the fully drawn yarn Yd measured by each tension sensor 5 is input to the controller 8.

The output device 7 is, for example, a display. In this case, the measurement result of the tension of the fully drawn yarn Yd by the tension sensor 5 is displayed on the output device 7 by the control of the controller 8. An operator can determine the dyeing characteristics of the fully drawn yarn Yd based on the measurement result output by the output device 7. Furthermore, the output device 7 may be, for example, a printer. In this case, the output device 7 prints the measurement result of the tension of the fully drawn yarn Yd by the tension sensor 5 on paper or the like. The tension measurement result may be transmitted to a terminal device or the like connected to the spun yarn take-up machine 1 via a network.

Here, a method for determining the dyeing characteristics of the fully drawn yarn Yd based on the measured value of the tension of the fully drawn yarn Yd will be described.

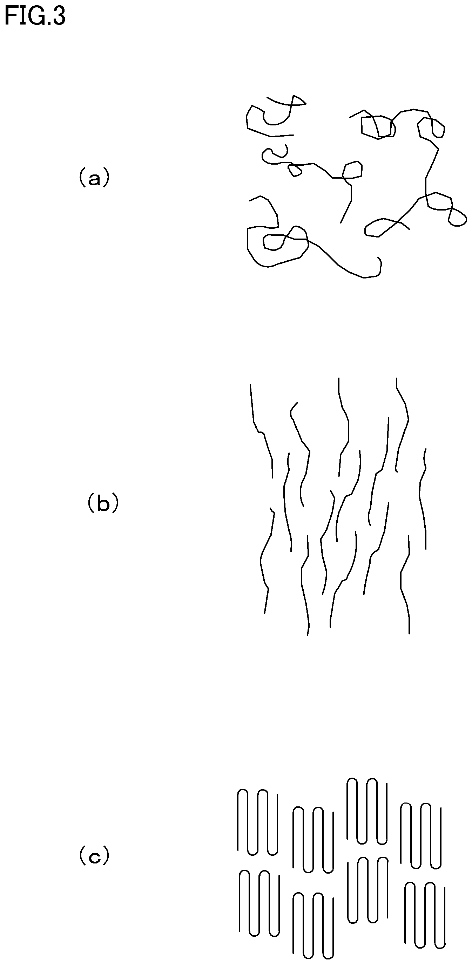

First, the molecular structure of the fully drawn yarn Yd will be described with reference to FIGS. 3A to 3C. The molecular structure of the fully drawn yarn Yd can be classified into three structures of a structure I (see FIG. 3A), a structure II (see FIG. 3B), and a structure III (see FIG. 3C).

As shown in FIG. 3A, the structure I is a non-oriented amorphous part in which molecules are neither oriented nor crystallized. In the non-oriented amorphous part, intermolecular gaps are large, and dyes easily enter. That is, the non-oriented amorphous part is easily dyed.

As shown in FIG. 3B, the structure II is an oriented amorphous part in which molecular orientation has progressed but crystallization has not yet occurred. Since the oriented amorphous part has almost no intermolecular gap, the dye hardly enters. That is, the oriented amorphous part is hardly dyed. In addition, the orientation amorphous part causes orientation relaxation to return to the non-oriented structure I by heating. As a result, the oriented amorphous part has a characteristic that shrinkage force increases during heating.

As shown in FIG. 3C, the structure III is an oriented crystalline part in which molecular orientation has progressed and crystallization has also progressed. Since the oriented crystalline part has almost no intermolecular gap, the dye hardly enters. That is, the oriented crystalline part is hardly dyed. In addition, since the oriented crystalline part has a strong molecular structure, the oriented crystalline part has a characteristic of increasing the resistance force at the time of stretching.

In view of the characteristics of the above three molecular structures (structure I, structure II, and structure III), the dyeing characteristics of the fully drawn yarn Yd can be determined based on the tension of the fully drawn yarn Yd measured by the tension sensor 5.

That is, the tension sensor 5 measures the tension of the fully drawn yarn Yd in the stretched state while being heated. Therefore, the measured value of the tension becomes larger for the fully drawn yarn Yd in which the proportion occupied by the oriented amorphous part having the characteristic that the shrinkage force increases during heating is higher. In addition, the measured value of the tension becomes larger for the fully drawn yarn Yd in which the proportion occupied by the oriented crystalline part having the characteristic that the resistance force at the time of stretching increases is higher. That is, it can be determined that the proportion occupied by the oriented amorphous part and the oriented crystalline part is higher in the fully drawn yarn Yd having a larger measured value of the tension than in the non-oriented amorphous part. Here, the oriented amorphous part and the oriented crystalline part are less likely to be dyed than the non-oriented amorphous part. Therefore, it can be determined that the fully drawn yarn Yd having a larger measured value of the tension has a characteristic of being less likely to be dyed.

Therefore, the dyeing density of the fully drawn yarn Yd can be determined from the average value of the tensions at a plurality of positions in the longitudinal direction of the fully drawn yarn Yd measured by the tension sensor 5. That is, it can be determined that the fully drawn yarn Yd having a larger average value of the measured values of the tension at a plurality of positions in the longitudinal direction of the fully drawn yarn Yd is less likely to be dyed and is dyed lighter.

The likelihood of the dyeing unevenness can be determined from variations in tension at a plurality of positions in the longitudinal direction of the fully drawn yarn Yd measured by the tension sensor 5. That is, in the fully drawn yarn Yd, it can be determined that the dyeing ease locally changes, that is, the dyeing unevenness is likely to occur in a portion where the measured tension is greatly different from the average value of the tensions at all positions where the tension measurement is performed.

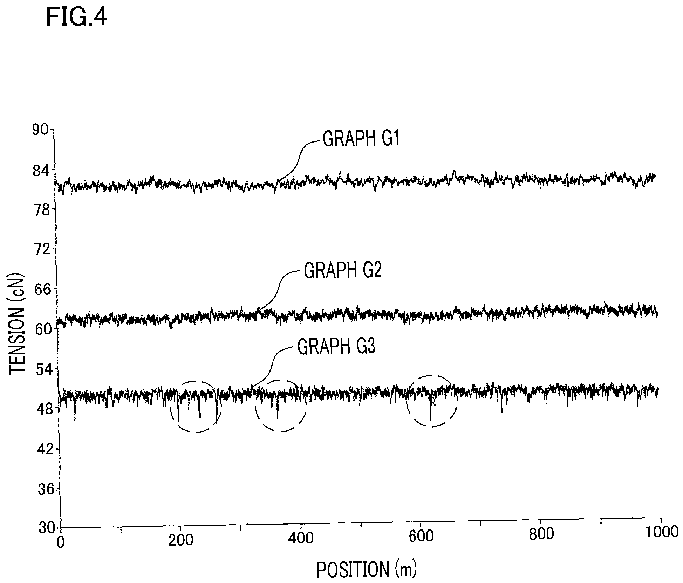

FIG. 4 shows the results of actually measuring the tensions of three types of fully drawn yarns Yd1, Yd2, and Yd3 that run while being heated at 120 °C to 140 °C and being stretched so that the deformation amount falls within the elastic range. A graph G1 is a graph relating to the fully drawn yarn Yd1, a graph G2 is a graph relating to the fully drawn yarn Yd2, and a graph G3 is a graph relating to the fully drawn yarn Yd3. The graphs G1, G2, and G3 show the relationship between the position in the longitudinal direction of the fully drawn yarn and the tension at that position. The measurement lengths are all 1000 m. The measurement was performed for each of the above-described yarns running 1000 meters.

The average value of the measured values of the tension of the fully drawn yarn Yd1 shown in the graph G1 is 81.1 cN. The average value of the measured values of the tension of the fully drawn yarn Yd2 shown in the graph G2 is 61.3 cN. The average value of the measured values of the tension of the fully drawn yarn Yd3 shown in the graph G3 is 49.4 cN. From this result, it can be predicted that the fully drawn yarn Yd1 having the largest average value of the measured values of the tension is dyed faintest. Furthermore, the fully drawn yarn Yd2 having the second largest average value of the measured values of the tension after the fully drawn yarn Yd1 can be predicted to be dyed slightly darker than the fully drawn yarn Yd1. Furthermore, it can be predicted that the fully drawn yarn Yd3 having the smallest average value of the measured values of the tension is dyed darkest.

Further, in the graph G3, there is a portion where the measured value of the tension greatly varies as compared with the graphs G1 and G2. That is, in the graph G3, there is a portion where the value of the tension is greatly different from the average value of the tension of the fully drawn yarn Yd3 (for example, portions surrounded by broken lines in FIG. 4). In this portion, it can be predicted that the dyeing ease locally changes. The portions surrounded by the broken lines in FIG. 4 have the tension much lower than the average value, so that it can be predicted that the portion is more likely to be dyed than the other portions. Therefore, it can be predicted that the fully drawn yarn Yd3 is more likely to cause dyeing unevenness than the fully drawn yarns Yd1 and Yd2.

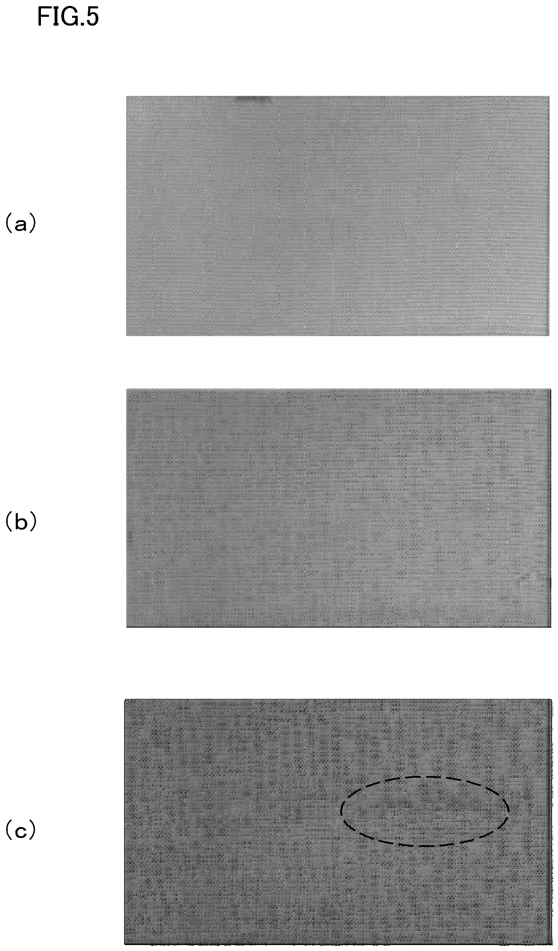

FIGS. 5A, 5B, and 5C show the results of dyeing samples S1, S2, and S3 produced by knitting the above-described fully drawn yarns Yd1, Yd2, and Yd3, respectively. FIG. 5A is a stained image of the sample S1 produced with the fully drawn yarn Yd1, FIG. 5B is a stained image of the sample S2 produced with the fully drawn yarn Yd2, and FIG. 5C is a stained image of the sample S3 produced with the fully drawn yarn Yd3. Each stained image is darker in the order of the sample S1, the sample S2, and the sample S3. That is, the fully drawn yarns Yd1, Yd2, and Yd3 are dyed darker in this order. Therefore, it was confirmed that the dyeing density of the fully drawn yarns Yd1, Yd2, and Yd3 matched the prediction based on the tension measurement result shown in FIG. 4.

In addition, no staining unevenness is found in the stained image of the sample S1 and the stained image of the sample S2. On the other hand, staining unevenness (for example, a portion surrounded by a broken line in FIG. 5C) is observed in the stained image of the sample S3. That is, the fully drawn yarn Yd3 is more likely to have uneven dyeing than the fully drawn yarns Yd1 and Yd2. Therefore, it was confirmed that the likelihood of the dyeing unevenness of the fully drawn yarns Yd1, Yd2, and Yd3 matched the prediction based on the tension measurement result shown in FIG. 4.

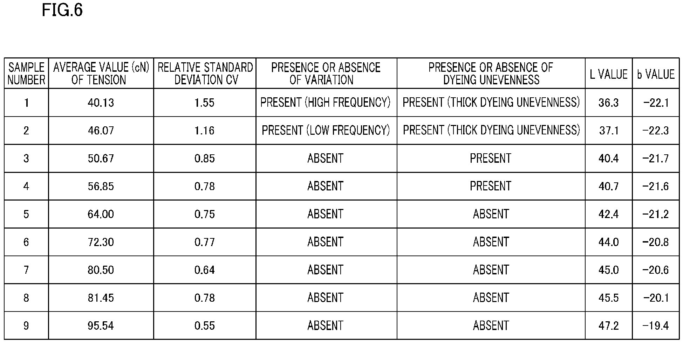

Furthermore, the table shown in FIG. 6 shows the tension measurement results and the confirmation results of the dyeing characteristics of the nine types of fully drawn yarns Yd (samples 1 to 9). In the tension measurement, similarly to that as described above, the tensions of the fully drawn yarns Yd (samples 1 to 9) running in a state of being stretched so that the deformation amount falls within the elastic range while being heated at 120 °C to 140 °C were measured. In the confirmation of the dyeing characteristics, similarly to that as described above, a sample produced by knitting each of the fully drawn yarns Yd (samples 1 to 9) was dyed, and visual inspection and color measurement were performed. As the tension measurement result, the average value of the measured values of the tension, the relative standard deviation (coefficient of variation) CV, and the presence or absence of variation of the measured values of the tension on the chart waveform are shown. As the confirmation result of the dyeing characteristics, the presence or absence of dyeing unevenness by visual observation, and the L value and the b value in color measurement are shown. Since blue staining was performed, the a value in color measurement is not relevant.

As shown in the table of FIG. 6, the average value of the measured values of the tension is larger for the fully drawn yarn having a larger sample number. The L value of the color measurement is larger for the fully drawn yarn having a larger sample number. Here, the larger the L value, the brighter the color. That is, the result that the fully drawn yarn having a larger sample number (fully drawn yarn having a larger average value of measured values of tension) was brighter (lighter) was obtained. The b values of the color measurement are all negative values, and the value is generally larger for the fully drawn yarn having a larger sample number. Here, when the b value is a negative value, the smaller the b value, the stronger the blueness. That is, the result that the blueness was weaker (lighter) for the fully drawn yarn having a larger sample number (fully drawn yarn having a larger average value of measured values of tension) was obtained.

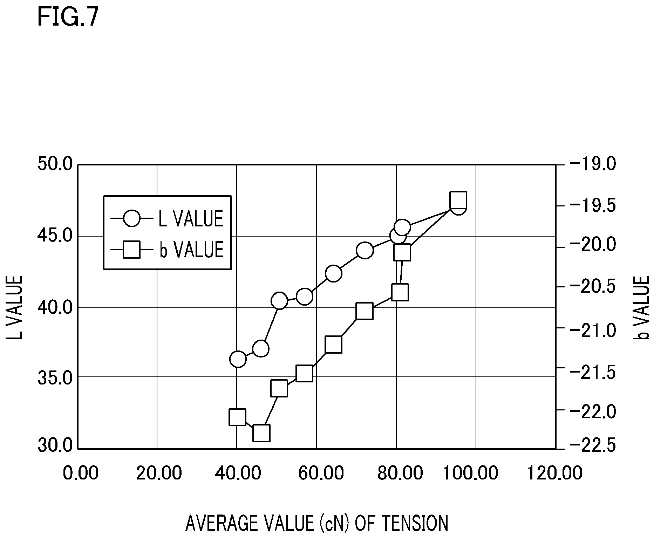

The graph shown in FIG. 7 shows the relationship between the average value of the measured values of the tensions of the fully drawn yarns Yd of the samples 1 to 9 and the L value and the b value. Also from the graph of FIG. 7, it can be seen that the L value is larger (lighter) for the fully drawn yarn having a larger average value of the measured values of the tension. In addition, it can be seen that the b value is larger (lighter) for the fully drawn yarn having a larger average value of the measured values of the tension.

Furthermore, as shown in the table of FIG. 6, in the fully drawn yarns of the samples 1 and 2 having a relatively large relative standard deviation CV, there is a variation in the chart waveform of the measured value of the tension, and thick dyeing unevenness occurs. In addition, even in the fully drawn yarns of the samples 3 and 4 having the second largest relative standard deviation CV after the fully drawn yarns of the samples 1 and 2, uneven dyeing occurred. In the fully drawn yarns of the samples 5 to 9 having a relatively small relative standard deviation CV, dyeing unevenness does not occur.

### (Features of First Embodiment)

As described above, the spun yarn take-up machine 1 of the present embodiment includes: the godet rollers 31 to 33 which are preliminary heating rollers that heat the yarn Y; and the godet rollers 34 and 35 which are heat-setting rollers that are arranged downstream in the yarn running direction of the godet rollers 31 to 33, heat the yarn Y at a higher temperature than the godet rollers 31 to 33, and feed the yarn Y at a yarn feeding speed higher than the yarn feeding speed of the godet rollers 31 to 33, and the spun yarn take-up machine 1 produces the fully drawn yarn Yd by subjecting the yarn Y spun from the spinning apparatus 2 to drawing processing. (i) The tension sensor 5 which is arranged between the guide roller 13 and the guide roller 14, which are two rollers adjacent in the yarn running direction, and which measures the tension of the yarn Y and (ii) the heater 6 that is arranged between the guide roller 13 and the guide roller 14 and heats the yarn Y are further provided. The tension sensor 5 measures the tension of the yarn Y subjected to the drawing processing between the godet roller 33, which is a preliminary heating roller, and the godet roller 34, which is a heat-setting roller, on the downstream in the yarn running direction of the godet roller 34 located on the most upstream in the yarn running direction of the heat-setting roller.

According to the configuration described above, the tension of the fully drawn yarn Yd in a state of being heated by the heater 6 can be measured by the tension sensor 5. Here, the oriented amorphous part in which the orientation in the fully drawn yarn Yd is advanced but not crystallized is less likely to be dyed than the non-oriented amorphous part in which the orientation and the crystallization are not performed. In addition, the oriented amorphous part has a characteristic that shrinkage force increases during heating. Therefore, it can be determined that the fully drawn yarn Yd having a larger tension measured in a heated state has a higher proportion occupied by the oriented amorphous part than the non-oriented amorphous part, and has a characteristic of being less likely to be dyed. That is, the dyeing characteristics of the fully drawn yarn Yd can be determined based on the tension of the yarn Y measured by the tension sensor 5 without requiring the step of actually dyeing the yarn Y. The tension sensor 5 provided in the apparatus for processing the fully drawn yarn Yd can acquire tension for determining the dyeing characteristics of the fully drawn yarn Yd in the process of processing the fully drawn yarn Yd. Therefore, the dyeing characteristics can be determined in a short time.

In the spun yarn take-up machine 1 of the present embodiment, the guide roller 14 is arranged downstream of the guide roller 13 in the yarn running direction, and the yarn feeding speed of the guide roller 14 is set to be faster than the yarn feeding speed of the guide roller 13. The tension sensor 5 measures the tension of the yarn Y in a state of being stretched between the guide roller 13 and the guide roller 14.

According to the configuration described above, the tension of the fully drawn yarn Yd in the stretched state can be measured by the tension sensor 5. Here, in addition to the oriented amorphous part, the oriented crystalline part in which molecular orientation in the fully drawn yarn Yd is advanced and crystallization is also advanced is less likely to be dyed than the non-oriented amorphous part. In addition, the oriented crystalline part has a characteristic that the resistance force increases at the time of stretching. Therefore, it can be determined that the fully drawn yarn Yd having a larger tension measured in the stretched state while being heated has a higher proportion occupied by the oriented amorphous part and the oriented crystalline part than the non-oriented amorphous part, and has a characteristic of being less likely to be dyed. Therefore, the dyeing characteristics of the fully drawn yarn Yd can be more accurately determined.

### <Second Embodiment>

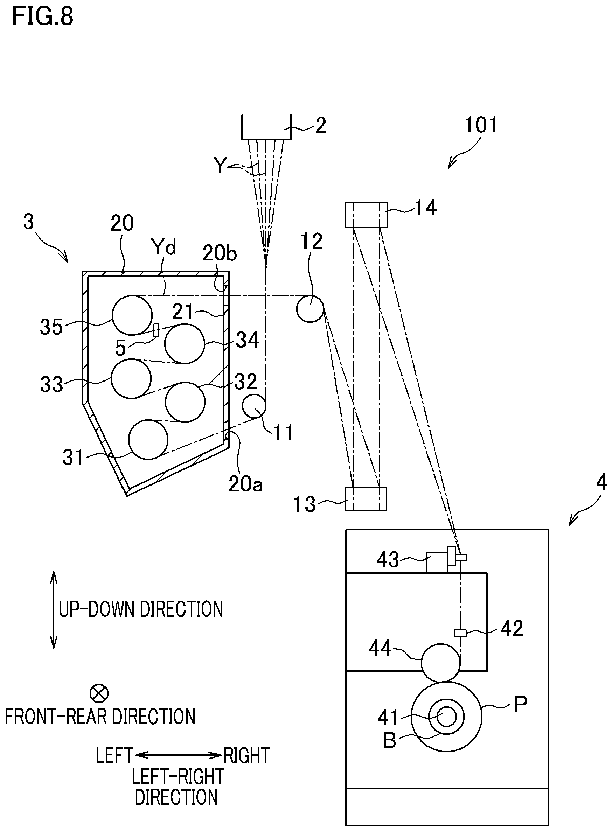

Next, a spun yarn take-up machine according to a second embodiment of the present invention will be described with reference to FIG. 8.

In the spun yarn take-up machine 1 of the first embodiment, the tension sensor 5 and the heater 6 are arranged between the guide roller 13 and the guide roller 14. On the other hand, in a spun yarn take-up machine 101 of the present embodiment, the tension sensor 5 is arranged between the godet roller 34 and the godet roller 35, and the heater 6 is not provided. Other configurations of the spun yarn take-up machine 101 of the present embodiment are the same as those of the spun yarn take-up machine 1 of the first embodiment, and thus a detailed description thereof will be omitted.

As described above, in the spun yarn take-up machine 101 of the present embodiment, the tension sensor 5 is arranged between the godet roller 34 and the godet roller 35, which are two rollers adjacent in the yarn running direction. The godet roller 34 and the godet roller 35 are both heat-setting rollers, and are capable of heating the yarn Y. Therefore, the godet roller 34 and the godet roller 35 correspond to "first roller and second roller" of the present invention.

The tension sensor 5 measures the tension of the fully drawn yarn Yd in a state of being heated by the godet roller 34 and the godet roller 35. The yarn feeding speeds of the godet roller 34 and the godet roller 35 are the same. Therefore, the fully drawn yarn Yd between the godet roller 34 and the godet roller 35 is not slackened and is not stretched. Therefore, the tension sensor 5 measures the tension of the fully drawn yarn Yd in a state of not being slackened and not being stretched.

Similarly to the first embodiment, the tension sensor 5 is arranged on each of the plurality of fully drawn yarns Yd running between the godet roller 34 and the godet roller 35. That is, the tensions of the plurality of fully drawn yarns Yd can be measured by the plurality of tension sensors 5, respectively.

### (Features of Second Embodiment)

In the present embodiment, the tension sensor 5 can measure the tension of the fully drawn yarn Yd in a state of being heated by the godet roller 34 and the godet roller 35. Therefore, similarly to the first embodiment, the dyeing characteristics of the fully drawn yarn Yd can be determined based on the tension of the fully drawn yarn Yd acquired in the process of processing the fully drawn yarn Yd. Therefore, the dyeing characteristics can be determined in a short time.

In the present embodiment, the godet roller 34 and the godet roller 35 are heat-setting rollers. According to this configuration, the tension sensor 5 can measure the tension of the yarn Y heated by the godet roller 34 and the godet roller 35, which are heat-setting rollers. Therefore, the tension of the yarn Y in the heated state can be measured without providing an extra roller for heating the yarn Y. Furthermore, the tension sensor 5 can measure the tension of the yarn Y reliably heated by the two heat-setting rollers.

Although the embodiments of the present invention have been described above with reference to the drawings, it should be understood that specific configurations are not limited to these embodiments. The scope of the present invention is defined not by the description of the above embodiments but by the claims, and includes meanings equivalent to the claims and all modifications within the scope.

In the first embodiment described above, the case where the tension sensor 5 and the heater 6 are arranged between the guide roller 13 and the guide roller 14 has been described, but the arrangement portions of the tension sensor 5 and the heater 6 are not limited thereto. That is, for example, the tension sensor 5 and the heater 6 may be arranged between the guide roller 12 and the guide roller 13. The tension sensor 5 and the heater 6 may be arranged downstream of the godet roller 34 in the yarn running direction.

In the first embodiment described above, the fully drawn yarn Yd is stretched by a difference in yarn feeding speed between the guide roller 13 and the guide roller 14, which are two rollers sandwiching the tension sensor 5. The tension sensor 5 measures the tension of the yarn Y in a state of being stretched between the guide roller 13 and the guide roller 14. However, the yarn feeding speeds of the two rollers sandwiching the tension sensor 5 are the same, and the tension sensor 5 may measure the tension of the yarn Y in a state of not being slackened and not being stretched.

In the second embodiment described above, the case in which the tension sensor 5 is arranged between the godet roller 34 and the godet roller 35, which are two heat-setting rollers, and the tension of the yarn Y heated by the godet roller 34 and the godet roller 35 is measured has been described, but the present invention is not limited thereto. That is, for example, the tension sensor 5 may be arranged between the godet roller 35 and the guide roller 12. In this case, the godet roller 35 arranged upstream in the yarn running direction among the godet roller 35 and the guide roller 12, which are two rollers sandwiching the tension sensor 5, is a heat-setting roller. Therefore, the tension sensor 5 can measure the tension of the yarn Y heated by the godet roller 35.

The tension sensor 5 may be capable of measuring the tension of the yarn Y heated by a roller other than the heat-setting roller. That is, for example, the tension sensor 5 may be arranged between the guide roller 12 and the guide roller 13, and the guide roller 12 located at least on the upstream in the yarn running direction among the guide roller 12 and the guide roller 13 may be a roller capable of heating the yarn Y.

In the second embodiment described above, the yarn feeding speeds of the godet roller 34 and the godet roller 35, which are two rollers sandwiching the tension sensor 5, are the same. The tension sensor 5 measures the tension of the yarn Y in a state of not being slackened and not being stretched. However, the fully drawn yarn Yd may be stretched by a difference in yarn feeding speed between the two rollers sandwiching the tension sensor 5, and the tension sensor 5 may measure the tension of the yarn Y in the drawn state.

In the first and second embodiments described above, the case where the three godet rollers 31 to 33 as the preliminary heating rollers and the two godet rollers 34 and 35 as the heat-setting rollers are provided has been described, but the present invention is not limited thereto. That is, at least one preliminary heating roller and at least one heat-setting roller may be provided.

## Claims

1. An apparatus (101) for producing a fully drawn yarn comprising:
a preliminary heating roller (31 to 33) that heats a yarn (Y); and
at least one heat-setting roller (34, 35) that is arranged downstream in a yarn running direction of the preliminary heating roller (31 to 33), heats the yarn (Y) at a higher temperature than the preliminary heating roller (31 to 33), and feeds the yarn (Y) at a yarn feeding speed higher than a yarn feeding speed of the preliminary heating roller (31 to 33),
the apparatus being configured to produce a fully drawn yarn by subjecting a yarn (Y) spun from a spinning apparatus (2) to drawing processing,
the apparatus comprising:
a plurality of feed rollers (12 to 14 and 31 to 35) that include the preliminary heating roller (31 to 33) and the at least one heat-setting roller (34, 35) and feed the yarn (Y); and
a tension sensor (5) that is arranged between a first roller (34) and a second roller (35), which are two feed rollers adjacent in the yarn running direction among the plurality of feed rollers (12 to 14, 31 to 35), and measures a tension of the yarn (Y),
wherein at least one of the first roller (34) and the second roller (35) located upstream in the yarn running direction is capable of heating the yarn (Y), and
wherein the tension sensor (5) measures a tension of the yarn (Y) subjected to drawing processing between the preliminary heating roller (31 to 33) and a most upstream heat-setting roller (34) downstream in the yarn running direction of the most upstream heat-setting roller (34) located the most upstream in the yarn running direction of the at least one heat-setting roller (34, 35).

2. The apparatus (101) for producing a fully drawn yarn according to claim 1, wherein one of the first roller (34) and the second roller (35) arranged upstream in the yarn running direction is the heat-setting roller (34).

3. The apparatus (101) for producing a fully drawn yarn according to claim 2,
wherein at least two heat-setting rollers (34, 35) are provided, and
wherein the first roller (34) and the second roller (35) are the heat-setting rollers (34 and 35).

4. An apparatus (1) for producing a fully drawn yarn comprising:
a preliminary heating roller (31 to 33) that heats a yarn (Y); and
at least one heat-setting roller (34, 35) that is arranged downstream in a yarn running direction of the preliminary heating roller (31 to 33), heats the yarn (Y) at a higher temperature than the preliminary heating roller (31 to 33), and feeds the yarn (Y) at a yarn feeding speed higher than a yarn feeding speed of the preliminary heating roller (31 to 33),
the apparatus being configured to produce a fully drawn yarn by subjecting a yarn (Y) spun from a spinning apparatus (2) to drawing processing,
the apparatus comprising:
a plurality of feed rollers (12 to 14 and 31 to 35) that include the preliminary heating roller (31 to 33) and the at least one heat-setting roller (34, 35) and feed the yarn (Y);
a tension sensor (5) that is arranged between a first roller (13) and a second roller (14), which are two feed rollers adjacent in the yarn running direction among the plurality of feed rollers (12 to 14, 31 to 35), and measures a tension of the yarn (Y); and
a heater (6) that is arranged between the first roller (13) and the second roller (14) and heats the yarn (Y),
wherein the tension sensor (5) measures a tension of the yarn (Y) subjected to drawing processing between the preliminary heating roller (31 to 33) and the most upstream heat-setting roller (34) downstream in the yarn running direction of the most upstream heat-setting roller (34) located most upstream in the yarn running direction of the at least one heat-setting roller (34, 35).

5. The apparatus (1, 101) for producing a fully drawn yarn according to any one of claims 1 to 4,
wherein the second roller (14, 35) is arranged downstream of the first roller (13, 34) in the yarn running direction,
wherein a yarn feeding speed of the second roller (14, 35) is set to be higher than a yarn feeding speed of the first roller (13, 34), and
wherein the tension sensor (5) measures a tension of the yarn (Y) in a state of being stretched between the first roller (13, 34) and the second roller (14, 35).
